# EUROPEAN PATENT SPECIFICATION

(11) **EP 2 165 702 B1**
(45) Date of publication and mention of the grant of the patent: **16.11.2011**
(21) Application number: 08016396.7
(22) Date of filing: 17.09.2008
(51) Int. Cl.: A61K 9/16, A61K 9/20, A61K 31/41

(54) **Stable and readily dissolved compositions of candesartan cilexetil prepared with wet granulation**
Durch Nassgranulation hergestellte stabile und fertig gelöste Zusammensetzungen aus Candesartan-Cilexetil
Compositions dissoutes facilement et stables de candesartan cilexetil préparé avec une granulation par voie humide

(43) Date of publication of application: 24.03.2010
(73) Proprietor: Helm AG, 20097 Hamburg (DE); Bluepharma, Industria Farmaceutica, S.A., 3045-016 Coimbra (PT)
(72) Inventor: Gindullis, Frank, Dr., 25365 Klein Offenseth-Sparrieshoop (DE); Simoes, Sergio, 3000-145 Coimbra (PT); Leitão da Silva, Alberto Gabriel, 3030-181 Coimbra (PT)
(74) Representative: Becker, Eberhard

(56) References cited:
- WO-A-2005/070398
- WO-A-2005/079751
- WO-A-2006/113631
- WO-A-2008/065097
- WO-A-2008/077823
- WO-A-2008/109170

## Description

### FIELD OF THE INVENTION

The present invention relates to new pharmaceutical compositions in which Candesartan cilexetil is contained in a stabilized form with enhanced solubility and from which it is readily bioavailable when applied in conventional pharmaceutical dosage forms, and a method for preparing the same. The pharmaceutical compositions of Candesartan cilexetil or of its combinations with other active ingredients can be used in methods to treat subjects suffering from cardiovascular diseases.

### BACKGROUND OF THE INVENTION

Candesartan cilexetil is the INN for (±)-1-(cyclohexyloxy-carbonyloxy)ethyl 2-ethoxy-1-{[2'-(1H-tetrazol-5-yl)biphenyl-4-yl]methyl}-1H-benzimidazol-7-carboxylate, and can be represented by the following structural formula:

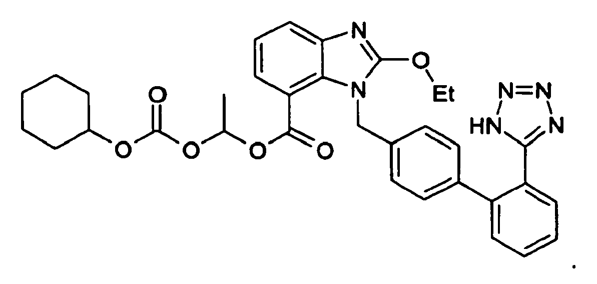

This compound is a widely employed non-peptide angiotensin-II receptor antagonist with a specific selectivity for AT1 receptors. As a pro-drug, the cilexetil derivative of Candesartan is a carrier, which is hydrolyzed by esterases to leave the active species Candesartan during resorption from the gastrointestinal tract. In this degradation, the 1-(cyclohexyloxy-carbonyloxy-ethyl) substituent is cleaved from the parent benzimidazol-7-carboxylic acid.

Like other benzimidazole-7-carboxylates, Candesartan exhibits a strong and particularly effective hypotensive action with reduced side effects as compared to other angiotensin-II receptor antagonists. Candesartan cilexetil is administered - in a relatively low daily dosage of less than 32 mg - as a single-substance preparation (mono-preparation), but also in combination with other active constituents, mainly diuretics such as hydrochlorothiazide, for the treatment of cardiovascular diseases. Moreover, Candesartan cilexetil is generally in extensive clinical use for a wide spectrum of indications (*see, for instance,* Arzneimittelwirkungen: Lehrbuch der Pharmakologie und Toxikologie, E. Mutschler, G. Geisslinger, H.K. Kroemer, M Schäfer-Korting, Herausgeber, 8. Auflage, Wissenschaftliche Verlagsgesellschaft mbH Stuttgart, 2001*).*

One of the main problems associated with the application of Candesartan cilexetil as an oral medication is the low bioavailability/solubility of the active ingredient, Candesartan. From clinical data it has been estimated that only between 15 and 40 % of the Candesartan contained in conventional drugs is becoming physiologically available and active.

Therefore, in drug formulation of Candesartan cilexetil it is of prime importance to secure the required reproducible bioavailability.

Bioavailability has two important aspects: 1. An active ingredient may have lower or higher intrinsic solubility in the respective physiological medium and at physiological temperature, as determined by thermodynamics. 2. It may have a slow or fast dissolution kinetics, such that solution equilibria are reached only slowly (asymptotically) or rapidly. It is obvious that for fast action high intrinsic solubility and fast dissolution kinetics are required.

For Candesartan cilexetil, several crystalline modifications (polymorphs) have been discovered, and an amorphous form is also available. Each of these forms has its intrinsic solubility and solubility kinetics. Moreover, it has been observed that the crystalline forms may be solvates (including hydrates) and that these polymorphs have different ranges of stability and can thus be interconverted. The amorphous form can be crystallized. In general, the neat amorphous form has been found to have a more limited stability towards temperature and hydrolytic or oxidative attack as compared to the crystalline polymorphs. However, in certain matrices it may be stabilized.

The second main problem associated with the application of Candesartan cilexetil is the limited stability of this compound, in particular in combination with excipients and other components. In the preparation of the currently available formulations some degradation, leading mainly to the des-ethyl compound, appears to be almost inevitable (see EP 0546 358 B1).

In the present invention it has been discovered that Candesartan cilexetil that is formulated according to the new process disclosed below has greatly improved stability and enhanced solubility compared to formulations prepared by direct compression methods known in the art.

### DESCRIPTION OF THE RELATED ART

Candesartan and Candesartan cilexetil were first introduced in EP 0 459 136 B1 and EP 0 720 982 B1: the process for the preparation of Candesartan cilexetil and its pharmaceutically acceptable salts is described in EP 0 459 136 B1, reference examples 2-8. The preparation of candesartan cilexetil in its C-type crystalline form and in its amorphous form is also described in EP 0 459 136 B1. Candesartan cilexetil of the C-type crystalline form (Form C) is obtained by crystallization from lower alcohols (methanol, ethanol), mixtures of a lower alcohol and water, and mixtures of a lower alkyl ketone (like acetone) and water.

Conventional formulations of drugs (and pro-drugs) including, among others, Candesartan cilexetil have hitherto included preparations where the active components are contained in oily or low-melting excipients, which allow a convenient production of tablets and related forms for oral administration: (see EP 0 546 358 B1, EP 0 782 852 B1 and EP 1 468 683 A1).

European Patent Application EP 1 997 479 (Helm AG) describes stabilized amorphous candesartan cilexetil compositions for oral administration comprising amorphous candesartan cilexetil and/or its solvates or hydrates, a stabilizing agent and optionally at least one pharmaceutically suitable excipient, which is insoluble or poorly soluble in organic solvents which have enhanced dissolution and reduced degradation characteristics.

EP 1 952 806 Al (Helm AG) relates to a process for the preparation of adsorbates of candesartan cilexetil and/or of its hydrates, wherein one starts from a solution consisting of candesartan cilexetil and/or its hydrates in at least one organic solvent, disperses in it an adsorbing material and removes the solvent and wherein the absorbing materials are selected in particular from the group of carbohydrates and related polyols, including celluloses, cellulose derivatives, sugars, sugar derivatives, cyclodextrins, starches, starch derivatives, polydextroses, or mixtures thereof.

In other formulations for oral application, active substances (including i. a. Candesartan, Candesartan cilexetil) are combined with surface-active excipients, e. g. sodium laurylsulfate in combination with taurine derivatives, which are believed to improve the absorption of the drugs (see EP 1 407 785 A1).

For liquid active ingredients, formulations with amphiphilic excipients, such as certain waxes, have been described which may also contain other active components. Candesartan is mentioned only as one of many pharmaceutically active compounds treated in this way (see EP 1 441 705 A1).

Formulations have also been based on dispersions of active ingredients with low solubility (including Candesartan) in lipophilic polymers which may also contain a wetting agent (see EP 1 592 406 A3).

Stable formulations of Candesartan cilexetil for oral application were obtained by introducing co-solvents (propyleneglycol and its esters, glycerin etc.) and fatty acid esters (glyceryl palmitate, caprate and oleate etc. (see WO 05/070398 A3)).

Candesartan cilexetil is also contained in stable formulations for oral application obtained in combinations with fatty acids (oleic and stearic acid etc.) or their esters (glyceryl caprate etc.), but also with phospholipids like lecithin (see EP 1 711 168 A3).

Candesartan cilexetil has similarly been integrated in stable formulations for oral application obtained by using water-soluble polymers like polyvinylalcohol, maltodextrine, xanthane etc. (see WO 05/084648 A1).

For a number of active ingredients with low solubility characteristics, granulates were obtained based on aqueous lactose as a binder. Candesartan has been mentioned non-specifically as one of those active ingredients which may be formulated following this process (see EP 1 793 801 A1).

In another formulation, Candesartan cilexetil has been integrated into hydrophilic colloids, like Carrageenan, as hydrophilic excipients (see EP 1 843 754 A1).

Solid dispersions of Candesartan and related All-antagonists for oral application have been obtained by applying special solubilizing agents, for which stearyl macrogel glycerides (Gelucire^{®}) serve as a prominent example. The formulations - made by applying a melt granulation process - showed enhanced bioavailabiliy of the drugs (see WO 06/113631 A3).

Improved formulations of hydrophobic active ingredients, including Candesartan, were prepared using particle-separating excipients, for which Poloxamer is a typical example (see WO 07/077581 A2).

Nanoparticulate Candesartan (or Candesartan cilexetil) formulations (particle size < 2000 nm) were obtained with a complex set of excipients, including polyols like hypromellose, sucrose, lactose, silicified microcrystalline cellulose, surface stabilizing and surface active ingredients, as well as lubricants like magnesium stearate, and others: (see WO 06/074218 A2).

### DESCRIPTION AND SUMMARY OF THE INVENTION

### Objective of the Invention

The objective of the studies and experiments leading to the present invention has been the design and preparation of conventional pharmaceutical dosage forms of Candesartan cilexetil, in which the active ingredient (Candesartan cilexetil) has both increased stability and enhanced bioavailability/solubility as compared to existing formulations. The formulations should also be conveniently accessible using procedures as established in general pharmacy. Unnecessary solvents should be avoided.

### MEANS FOR SOLVING THE ABOVE OBJECTIVES

Accordingly, a first aspect of the present invention is a method for preparing a wet granulated composition of Candesartan cilexetil and/or its solvates or hydrates comprising:
a) combining Candesartan cilexetil or its solvates or hydrates with an aqueous solution of sodium docusate as a first surfactant, and with sodium lauryl sulphate as a second surfactant, so that Candesartan cilexetil is homogeneously dispersed in the solution;
b) combining said dispersion from step (a) with at least one pharmaceutical excipient, preferably lactose and/or pregelatinized starch and/or calcium carmellose and/or hydroxypropylcellulose, under shear to generate wet granules;
c) optionally screening and sieving said wet granules;
d) drying said granules;
e) optionally mixing in other excipients; and
f) optionally forming the composition into unit dosage forms.

A second aspect of the present invention is a wet granulated pharmaceutical composition of Candesartan cilexetil and/or its solvates or hydrates, prepared by the above mentioned method, comprising:
a) Candesartan cilexetil or its solvates or hydrates, pretreated with an aqueous solution of sodium docusate as a first surfactant, and sodium lauryl sulphate as a second surfactant; and
b) at least one pharmaceutically suitable excipient.

A third aspect of the invention is the unit dosage form prepared from the wet granulated composition of Candesartan cilexetil. Preferably this unit dosage form is a tablet or capsule and wherein said dosage form (tablet) is preferably coated. The coat can be in the form of a quick dissolving film or polymer or can be a functional coat.

A fourth aspect of the invention is the combination of other additional active drugs in the same unit dosage form with said wet granulated compositions of Candesartan cilexetil. Preferably said other additional drug is selected from the groups of diuretics, preferably hydrochlorothiazide, of anti-hypertonics agents, preferably amlodipine and its salts and of reductase inhibitors, preferably Atorvastatin and its salts.

Said other active drug can be combined with the composition containing candesartan cilexetil by several methods. It is possible to add said other active drug to the solution of step (a) and/or to add to the mixture of step (b) and/or to add to the granules of step (e) to combine the composition with said other active drug.

### EFFECT OF THE INVENTION

It was surprisingly found that preparation of a dispersion of Candesartan cilexetil in an aqueous solution with even a low concentration of sodium docusate (1:6 [w/w] relative to Candesartan cilexetil) prior to its addition to a mixture of pharmaceutically acceptable excipients results in an enhanced solubility/bioavailability and stability of the drug as compared to other formulations obtained by methods commonly used for the preparation of solid dosage forms of Candesartan cilexetil.

### DESCRIPTION OF THE FIGURES:

The present invention is described in more detail by the following figures which show:
- Figure 1:: Flow chart of the process leading to the products of the present invention following the procedure described in Example 1.
- Figure 1a:: Dissolution vs. time diagram of the product obtained according to Example 1 and of the commercial product.
- Figure 2:: Flow chart of the preparation of a comparative product (Example 2).
- Figure 2a:: Dissolution vs. time diagram of the comparative product and a commercial product.
- Figure 3:: Release of drug (Candesartan cilexetil) from the products obtained according to Example 1 of the present invention and a comparative material obtained by direct compression (70 N).
- Figure 4:: Impurity profiles of the product of Example 1, of the comparative product of Example 2, of the *pure* active pharmaceutical ingredient (API = Candesartan cilexetil) and of a commercial product (Atacand^{®} 8 mg) detected after 0,15 and 30 days: a) Des-ethyl Candesartan cilexetil b) Unknown impurity at RRT 1.09 (see text) c) Total impurities

The analytical methods employed were as follows:

### Dissolution profiles:

According to Ph. Eur. 2.9.3 and USP <711> - Method 2 (Paddle Apparatus)

| **Medium** | Phosphate buffer pH 6.5 + 0,35% Tween 20 |
|---|---|
| **Volume** | 900 ml |
| **Temperature** | 37.0 °C ± 0.5 °C |
| **Method** | UV |
| **Stirring speed** | 50 rpm |

### Impurity profiles:

Chromatographic conditions:

| | |
|---|---|
| Column: | Zorbax SB-CN |
| Detection: | UV 210 nm. |
| Mobile phase: | Mobile Phase A - phosphate buffer 20mM, pH 3.0 |
| | Mobile Phase B: acetonitrile |

### Description of the Invention

In preliminary work it has been observed that Candesartan cilexetil (API) is released only very slowly and incompletely from any of the conventional tablets. It was then surprisingly found that treatment of the API with an aqueous solution of the wetting agent (or surfactant) sodium docusate greatly enhances and improves the release of the active constituent.

Moreover, the treatment with sodium docusate also clearly affected the stability of the formulations very significantly, leading to a product which is more robust against thermal and chemical degradation. This is true in particular regarding the formation of the des-ethyl derivative and of an unknown impurity with a relative retention time of 1.09. Even small impurities of these compounds are pharmaceutically unacceptable.

In this process, the active pharmaceutical ingredient (API; Candesartan cilexetil) is first homogeneously suspended in an aqueous solution of sodium docusate. This dispersion is screened by filtration and subjected to a wet granulation together with common excipients such as lactose, starch, calcium-carmellose, and/or hydroxyethylcellulose etc.. Sodium lauryl sulfate is added to reduce the hardness of the tablets (Example 1). The product is finally dried at 30-35°C and the dried granulate material is sieve-classified and coated with magnesium stearate as a lubricant. The product obtained can be pressed to tablets which are comparable in their pharmacotechnical properties to established commercial products. The concentration of API in the individual tablet (4, 8, 16, or 32 mg) can be adjusted by the relative amount of Candesartan cilexetil introduced into the primary mixture with sodium docusate and the excipients or by changing the tablet weight.

The advantage of the products obtained according to this process is the increased and enhanced solubility of the Candesartan cilexetil contained therein as compared to products of direct compression of neat Candesartan cilexetil or of Candesartan cilexetil with other excipients as described in the prior art.

The products obtained according to the present invention are also more chemically stable as demonstrated in stability test experiments.

Finally, the products can be prepared at low cost, without usage of any solvent other than water, and by employing standard equipment of the pharmaceutical industry.

The present invention discloses a wet granulation process which provides formulations of Candesartan cilexetil in a stable form which contain significantly reduced amounts of impurities (decomposition and degradation products).

In the formulation of Candesartan cilexetil in a wet granulation process, combinations of diluents, binders, disintegrants, lubricants and other additives known in the art are used to provide the properties needed for the unit dosage form as is known in the art. For example, for preparation of tablets, the combination provides for adequate tablet hardness upon compression while providing rapid disintegration in vivo. Although there is a wide degree of latitude in formulating Candesartan cilexetil to meet these conditions, typically such tablet formulations contain about 1-40% weight:weight (w:w) drug (pretreated with the surfactants), about 1-15% disintegrant, about 0-10% binder and about 0.1 - 4 % lubricant, with the bulk comprising a diluent and/or other components. Preferred binders include carboxymethylcellulose, hydroxyethylcellulose, hydroxypropylcellulose, dextrin, gelatine, guar gum, hydroxypropyl methylcellulose, maltodextrine, methylcellulose, polyethylene oxide, polymethacrylates and sodium alginate; a particularly preferred binder is hydroxypropylcellulose. A preferred lubricant is magnesium stearate. Preferred diluents include calcium phosphate, calcium sulfate, cellulose acetate, dextrates, dextrine, dextrose, fructose, kaolin, lactitol, lactose, maltitol, maltodextrine, maltose, microcrystalline cellulose, polymethacrylates, powdered cellulose, silicified microcrystalline cellulose, sodium chloride, sorbitol, sucrose and talc.

In preparation of Candesartan cilexetil compositions by wet granulation, any technique known in the art for wet granulation can be used for the purposes of the present invention (e. g. also spray granulation and optionally also spray drying)

An important element to these processes is that the granulation solvent containing the dispersed Candesartan cilexetil is added while the powder blend is under shear. The shear serves to break up incipient clumps and thereby provides a more uniform granulation. Non-limiting examples of shearing processes include high shear wet granulations, fluid-bed granulations, extrusion granulations and low shear wet granulations (such as stirrers, mixers and blenders, including bin blenders). The amount of wet granulation solvent added is determined based on adequate wetting to bind the majority of the fine particles.

The wet granulation solvent addition can be carried out using any technique known in the art. For example, the liquid can be added in single or multiple rapid additions, sprayed onto a stirring powder bed, pumped directly onto the powder or introduced into the fluidizing gas. Mixing times with the liquid are generally optimized such that the majority of fine particles are bound in granules, yet the granules themselves are not over-hardened.

Once the granules are formed, it is sometimes advantageous to grind and/or sieve the material while it is wet (softened), as is known in the art. The wet composition is preferably dried before use in formation of unit dosage forms. Such drying can be accomplished using any method known in the art. Non-limiting examples of these methods include air drying, fluid bed drying, microwave drying, oven drying, radio frequency drying, vacuum oven drying and convection oven drying. We have found that the control of the drying temperature is important to provide low levels of Candesartan cilexetil impurities. Preferably the drying temperature does not exceed about 60°C; more preferably, the temperature does not exceed about 50°C; most preferably, the temperature does not exceed about 35 °C. Once the granules are dry, it is sometimes desirable to adjust the particle size by grinding and/or sieving, as is known in the art. After this point, a lubricant is typically added followed by a short (about 1-10 minute) mixing period, typically carried out in a low shear blender such as a tumbling blender. Examples of said tumbling blenders include bin-blenders, V-blenders and Turbula™ blenders. The preferred lubricant is magnesium stearate. Once the blend is made, unit dosage forms are prepared by procedures known in the art. Preferably the unit dosage forms include tablets or capsules. Tablets are made by filling a die with the Candesartan cilexetil containing composition, then pressing with a matching punch. Capsules are prepared by filling shaped capsule shells, and then sealing. Such operations are preferably carried out using a rotary tablet press or commercial capsule-filling machine.

When Candesartan cilexetil is prepared in the form of a tablet, a disintegrant is desirable to provide for rapid disintegration of the tablet in the gastrointestinal tract and thereby assure that the drug is rapidly available for absorption. A large number of disintegrants are disclosed in the prior art for use with Candesartan cilexetil. For example, the following list of disintegrants is disclosed in International Patent Publication Number WO 03/011283A1 in combination with a second active pharmaceutical ingredient: calcium carboxymethylcellulose, sodium carboxymethylcellulose, silica, croscarmellose sodium, crospovidone, guar gum, magnesium aluminum silicate, methylcellulose, polacrilin potassium, cellulose, pregelatinized starch, sodium alginate, sodium starch glycolate and starch.

Preferred disintegrants for compositions useful for wet granulation of Candesartan cilexetil include starches, starch derivatives, powdered cellulose and cross-linked cellulose, and polyvinylpyrrolidone.

Particularly preferred starches include cornstarch and pregelatinized starch. These disintegrants are preferably used in compositions of Candesartan cilexetil at levels between about 1 and about 20% (w:w) of the overall formulation; more preferably between about 8 and about 20% (w:w).

Regarding the combination of Candesartan Cilexetil with other active pharmaceutical ingredients in the formulations prepared according to the present invention, additions of diuretics (such as hydrochlorothiazide), anti-hypertensive agents (such as Amlodipine and its salts) or HMG-CoA-reductase inhibitors (such as atorvastatin) are particularly prominent examples. Depending on their solubility, miscibility and stability, these additional components may be added to the liquid supporting the wet granulation, to the blend of excipients subjected to granulation, or to the granulation products.

### Example 1:

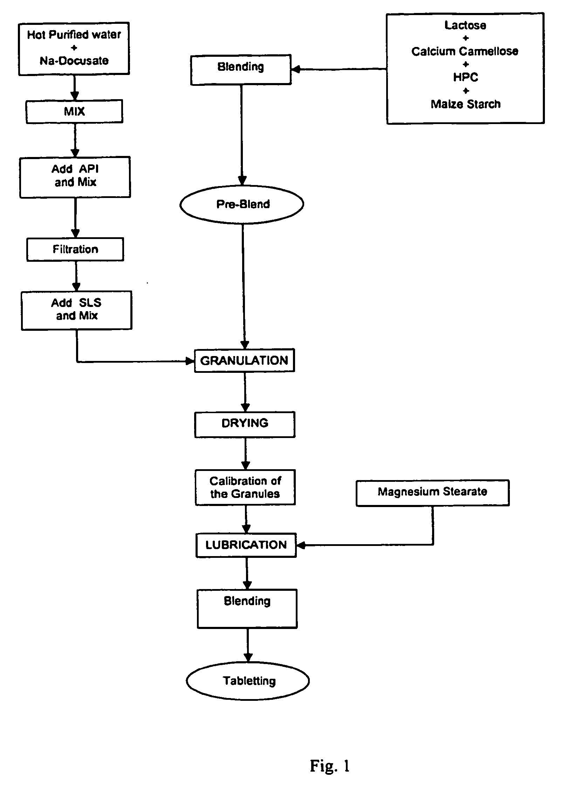

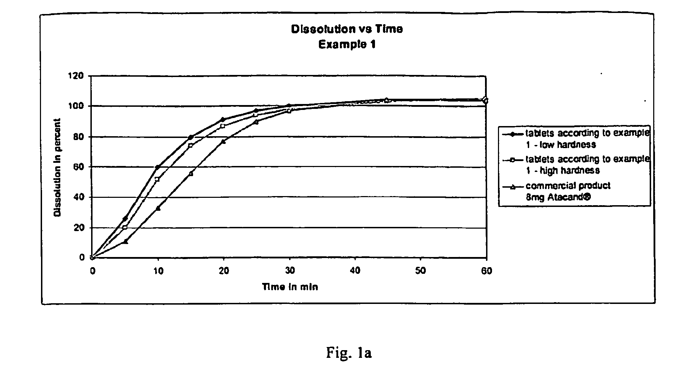

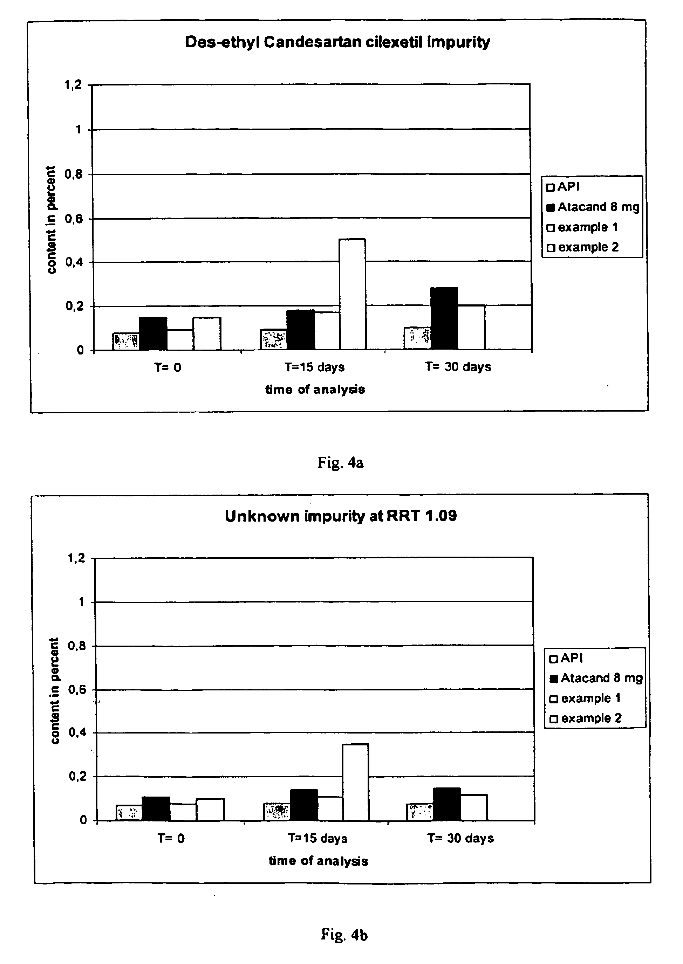

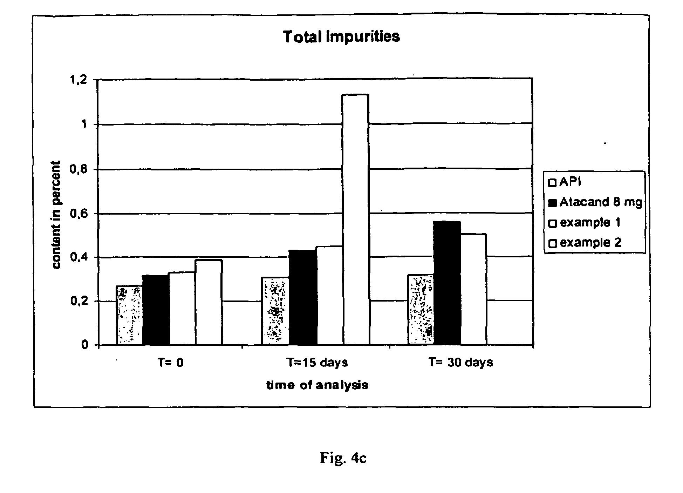

In the standard procedure according to the present invention, the ingredients shown in Table 1 are used and processed according to the flow-chart shown in Figure 1. Tablets with a total weight of 130 mg prepared from this product thus contain 8 mg of the active pharmaceutical ingredient and can be compared directly with a commercial product (see Figure 1a and 4a-4c). Dissolution vs. time diagrams of the product and of commercial material are shown in Figure 1a.

**Table 1**

| **Substance** | **Percentage** |
|---|---|
| Candesartan cilexetil | 6.2% |
| Calcium Carmellose, Ph. Eur | 3.0% |
| Hydroxypropylcellulose, Ph.Eur | 3.5% |
| Lactose Monohydrate, Ph. Eur | 68.3% |
| Pre-Gel Maize Starch, Ph.Eur | 16.7% |
| Sodium Docusate, Ph.Eur | 1.0% |
| Sodium laurylsulphate, Ph. Eur | 1,0% |
| Magnesium stearate, Ph.Eur | 0.3% |
| **total** | **100.0%** |

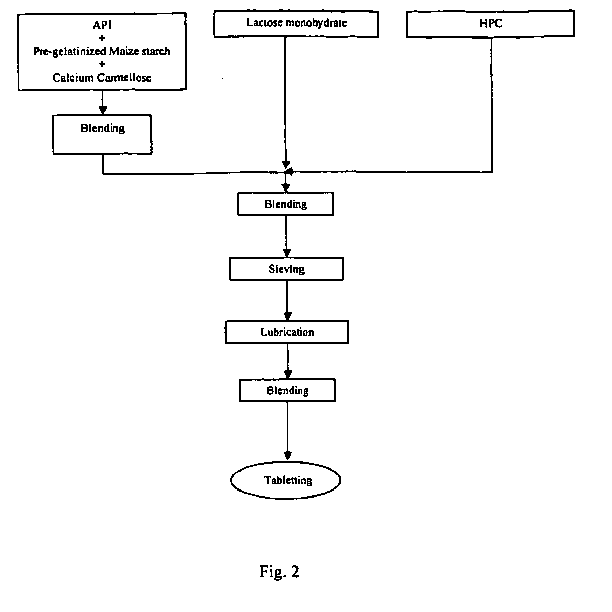

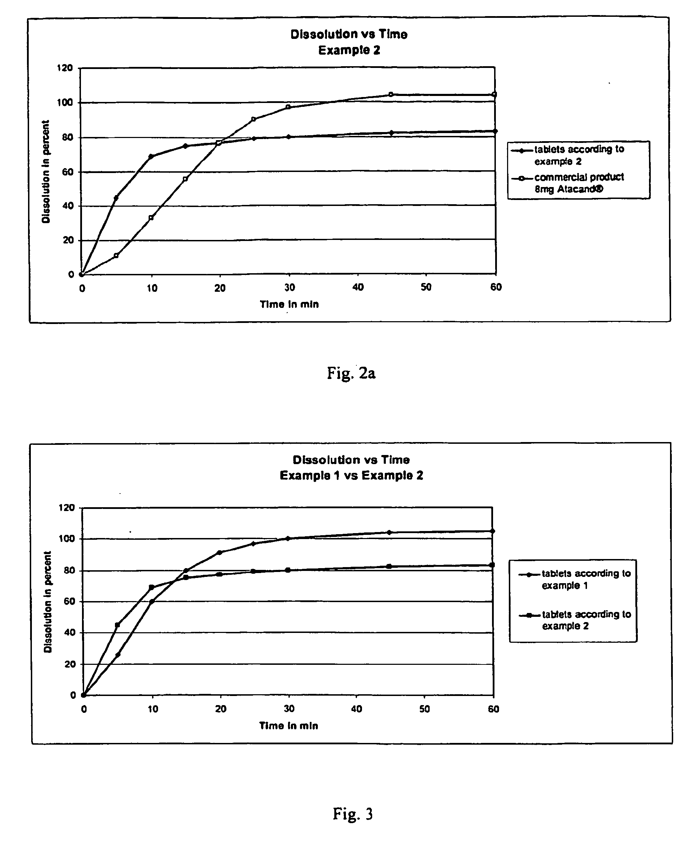

Example 2 (comparative Example):For the preparation of a comparative material (130 mg tablets with 8 mg Candesartan cilexetil) containing only standard excipients (no sodium docusate, no sodium lauryl sulphate, direct compression) the ingredients shown in Table 2 were used and processed according to the flow-chart shown in Figure 2. Figures 2a and 3 compare the dissolution vs. time diagrams of the products of the examples 1 and 2 and the commercial product, respectively..

**Table 2**

| **Substance** | **Percentage** |
|---|---|
| Candesartan cilexetil | 6.2% |
| Calcium Carmellose, Ph.Eur | 8.0% |
| Hydroxypropylcellulose, Ph.Eur | 5.5% |
| Lactose Monohydrate, Ph.Eur | 65.0% |
| Pre-Gel Maize Starch, Ph.Eur | 15.0% |
| Magnesium stearate, Ph.Eur | 0.3% |
| **total** | **100.0%** |

The stability of the products of Examples 1-2 and of commercial materials (pure API = Candesartan cilexetil, and Atacand 8 mg, a commercial sample) was monitored by chromatographic analysis over a period of 30 days (Figures 4a-c). From the results it is obvious that
a) the formation of the two prominent decomposition products des-ethyl candesartan cilexetil and a species with RRT = 1.09 is only slightly increased for the products obtained following the process disclosed in this invention for Example 1 (shown in Fig. 1) as compared to pure API (without excipients);
b) the formation of these decomposition products is greatly enhanced for the product of Example 2, where the API has been formulated following the conventional process shown in Fig. 2.
c) the stability of the product according to Example 1 is at least comparable to or even better than that of commercial formulations (like Atacand 8mg).

### Example 3:

Tablets of 500 mg total weight containing 32 mg of Candesartan cilexetil and 40 mg of Atorvastatin are prepared following the process shown in Figure 1. Atorvastatin (or one of its salts) may be added either to the liquid supporting the granulation process, to the blend of powders of the excipients subjected to granulation, or to the granulated product.

**Table 3**

| **Substance** | **Percentage** |
|---|---|
| Candesartan cilexetil | 6.4% |
| Atorvastatin hemicalcium 1,5 H₂O | 8.7% |
| Microcrystalline Cellulose, Ph. Eur | 12.0% |
| Hydroxypropylcellulose, Ph.Eur | 4.5% |
| Lactose Monohydrate, Ph.Eur | 61.6% |
| Carmellose Sodium, Ph.Eur | 4.0% |
| Sodium Docusate, Ph.Eur | 1.5% |
| Sodium lauryl sulphate, Ph.Eur | 0.6% |
| Magnesium stearate, Ph.Eur | 0.7% |
| **total** | **100.0%** |

The obtained tablets can be film-coated by techniques known in the art.

## Claims

1. A method for preparing a wet granulated composition of Candesartan cilexetil and/or its solvates or hydrates comprising:
a) combining Candesartan cilexetil or its solvates or hydrates with an aqueous solution containing sodium docusate as a first surfactant, and with sodium lauryl sulfate as a second surfactant, so that Candesartan cilexetil is homogeneously dispersed in the solution;
b) combining said dispersion from step (a) with at least one pharmaceutical excipient, preferably lactose and/or pregelatinized starch and/or calcium carmellose and/or hydroxypropylccllulose, under shear to generate wet granules;
c) optionally screening and sieving said wet granules;
d) drying said granules;
e) optionally mixing in other excipients; and
f) optionally forming the composition into unit dosage forms.

2. The method according to claim 1, wherein an aqueous solution of the first surfactant is used, wherein said surfactant is present at a concentration between about 1 % to 20% (w:w), preferably between 2% to 8 % (w:w).

3. A method of preparing a unit dosage form containing Candesartan cilexetil and/or its solvates or hydrates and at least one other active drug, wherein the composition prepared according to the method of claims 1 and 2 is combined with at least one other active drug and optionally additional excipients.

4. The method according to claim 3, wherein said other active drug is added to the solution of step (a) and/or added to the mixture of step (b) and/or added to the granules of step (e) to combine the composition with said other active drug.

5. A wet granulated pharmaceutical composition of Candesartan cilexetil and/or its solvates or hydrates, comprising:
a) Candesartan cilexetil or its solvates or hydrates, pretreated with an aqueous solution of sodium docusate as a first surfactant, and sodium lauryl sulfate as a second surfactant; and
b) at least one pharmaceutically suitable excipient,
obtained by a method according to any of claims 1 to 4.

6. The wet granulated pharmaceutical composition according to claim 5, wherein the pharmaceutical acceptable excipient is selected from celluloses and cellulose derivatives, lactose, starches and starch derivatives, cyclodextrins, polydextroses or mixtures of said substances and is preferably selected from hydroxypropylcellulose, lactose and calcium carmellose and pregelatinized starch.

7. A unit dosage form prepared from the wet granulated composition according to claim 5 or 6, wherein said unit dosage form is a tablet or capsule, wherein preferably said dosage form is coated.

8. The wet granulated pharmaceutical composition according to claim 5 or 6, wherein said combination of surfactants is present in a range of concentrations between about 0.1% and 10 % (w:w) of said composition, preferably in the range between 0.5% to 5 % (w:w) of said composition.

9. The wet granulated pharmaceutical composition according to claim 5 or 6, wherein said composition also contains at least one active drug in addition to Candesartan cilexetil.

10. The wet granulated pharmaceutical composition according to claim 9, wherein said additional active drug is selected from the groups of diuretics, preferably hydrochlorothiazide, of antihypertensive agents, preferably amlodipine and its salts and of reductase inhibitors, preferably Atorvastatin and its salts.

## Patentansprüche

1. Verfahren zum Herstellen einer feuchtgranulierten Zusammensetzung von Candesartan-Cilexetil und/oder dessen Solvaten oder Hydraten, umfassend:
a) Kombinieren von Candesartan-Cilexetil oder dessen Solvaten oder Hydraten mit einer wässerigen Lösung, enthaltend Natriumdocusat als einem ersten oberflächenaktiven Stoff, und mit Natriumlaurylsulfat als einem zweiten oberflächenaktiven Stoff, so dass Candesartan-Cilexetil in der Lösung homogen dispergiert wird;
b) Kombinieren der Dispersion aus Schritt (a) mit mindestens einem pharmazeutischen Exzipienten, vorzugsweise Lactose und/oder prägelatinisierter Stärke und/oder Calcium-Carmellose und/oder Hydroxypropylcellulose, unter Scherung, um feuchtes Granulat zu erzeugen;
c) gegebenenfalls Abtrennen und Sieben des feuchten Granulats;
d) Trocknen des Granulats;
e) gegebenenfalls Mischen mit anderen Exzipienten; und
f) gegebenenfalls Bilden von Einzeldarreichungsformen aus der Zusammensetzung.

2. Verfahren nach Anspruch 1, wobei eine wässerige Lösung des ersten oberflächenaktiven Stoffs verwendet wird, wobei der oberflächenaktive Stoff mit einer Konzentration zwischen ungefähr 1 % bis 20 % (Gew. : Gew.), vorzugsweise zwischen 2 % bis 8 % (Gew. : Gew.), vorliegt.

3. Verfahren zum Herstellen einer Einzeldarreichungsform, enthaltend Candesartan-Cilexetil und/oder dessen Solvate oder Hydrate und mindestens einen anderen aktiven Inhaltsstoff, wobei die nach dem Verfahren der Ansprüche 1 und 2 hergestellte Zusammensetzung mit mindestens einem anderen aktiven Inhaltsstoff, und gegebenenfalls zusätzlichen Exzipienten, kombiniert wird.

4. Verfahren nach Anspruch 3, wobei der andere aktive Inhaltsstoff der Lösung von Schritt (a) zugesetzt wird und/oder der Mischung von Schritt (b) zugesetzt wird und/oder dem Granulat von Schritt (e) zugesetzt wird, um die Zusammensetzung mit dem anderen aktiven Inhaltsstoff zu kombinieren.

5. Feuchtgranulierte pharmazeutische Zusammensetzung von Candesartan-Cilexetil und/oder dessen Solvaten oder Hydraten, umfassend:
a) Candesartan-Cilexetil oder dessen Solvate oder Hydrate, vorbehandelt mit einer wässerigen Lösung von Natriumdocusat als einem ersten oberflächenaktiven Stoff, und Natriumlaurylsulfat als einem zweiten oberflächenaktiven Stoff; und
b) mindestens einen pharmazeutisch geeigneten Exzipienten,
erhalten nach einem Verfahren nach einem der Ansprüche 1 bis 4.

6. Feuchtgranulierte pharmazeutische Zusammensetzung nach Anspruch 5, worin der pharmazeutisch annehmbare Exzipient ausgewählt ist aus Cellulosen und CelluloseDerivaten, Lactose, Stärken und Stärke-Derivaten, Cyclodextrinen, Polydextrosen oder Mischungen von diesen Substanzen, und vorzugsweise ausgewählt ist aus Hydroxypropylcellulose, Lactose und Calcium-Carmellose und prägelatinisierter Stärke.

7. Einzeldarreichungsform, hergestellt aus der feuchtgranulierten Zusammensetzung nach Anspruch 5 oder 6, worin die Einzeldarreichungsform eine Tablette oder Kapsel ist, worin die Darreichungsform vorzugsweise beschichtet ist.

8. Feuchtgranulierte pharmazeutische Zusammensetzung nach Anspruch 5 oder 6, worin die Kombination von oberflächenaktiven Stoffen in einem Konzentrationsbereich zwischen ungefähr 0,1 % und 10 % (Gew. : Gew.) der Zusammensetzung vorliegt, vorzugsweise in dem Bereich zwischen 0,5 % bis 5 % (Gew. : Gew.) der Zusammensetzung.

9. Feuchtgranulierte pharmazeutische Zusammensetzung nach Anspruch 5 oder 6, worin die Zusammensetzung ferner mindestens einen aktiven Inhaltsstoff zusätzlich zu Candesartan-Cilexetil enthält.

10. Feuchtgranulierte pharmazeutische Zusammensetzung nach Anspruch 9, worin der zusätzliche aktive Inhaltsstoff ausgewählt ist aus den Gruppen von Diuretika, vorzugsweise Hydrochlorothiazid, von blutdrucksenkenden Mitteln, vorzugsweise Amlodipin und dessen Salzen, und von Reduktasehemmern, vorzugsweise Atorvastatin und dessen Salzen.

## Revendications

1. Procédé de préparation d'une composition granulée humide de Candésartan cilexétil et/ou de ses solvates ou hydrates, comprenant :
a) la combinaison de Candésartan cilexétil ou de ses solvates ou hydrates avec une solution aqueuse contenant du docusate de sodium en tant que premier tensioactif, et avec du laurylsulfate de sodium en tant que second tensioactif, de sorte que le Candésartan cilexétil soit dispersé de manière homogène dans la solution ;
b) la combinaison de ladite dispersion de l'étape (a) avec au moins un excipient pharmaceutique, de préférence le lactose et/ou l'amidon prégélatinisé et/ou la carmellose calcique et/ou l'hydroxypropylcellulose, sous cisaillement pour générer des granules humides ;
c) le criblage et le tamisage facultatifs desdits granules humides ;
d) le séchage desdits granules ;
e) le mélange facultatif avec d'autres excipients ; et
f) la formation facultative de la composition en formes posologiques unitaires.

2. Procédé selon la revendication 1, dans lequel une solution aqueuse du premier tensioactif est utilisée, ledit tensioactif étant présent à une concentration comprise dans la plage allant d'environ 1 % à 20 % (p/p), de préférence comprise dans la plage allant de 2 % à 8 % (p/p).

3. Procédé de préparation d'une forme posologique unitaire contenant du Candésartan cilexétil et/ou ses solvates ou hydrates et au moins un autre principe actif, dans lequel la composition préparée selon le procédé des revendications 1 et 2 est combinée à au moins un autre principe actif et facultativement à d'autres excipients.

4. Procédé selon la revendication 3, dans lequel ledit autre principe actif est ajouté à la solution de l'étape (a) et/ou ajouté au mélange de l'étape (b) et/ou ajouté aux granules de l'étape (e) afin de combiner la composition audit autre principe actif.

5. Composition pharmaceutique granulée humide de Candésartan cilexétil et/ou de ses solvates ou hydrates, comprenant :
a) le Candésartan cilexétil ou ses solvates ou hydrates, prétraité(s) avec une solution aqueuse de docusate de sodium en tant que premier tensioactif, et de laurylsulfate de sodium en tant que second tensioactif ; et
b) au moins un excipient pharmaceutiquement acceptable,
obtenue par un procédé selon l'une quelconque des revendications 1 à 4.

6. Composition pharmaceutique granulée humide selon la revendication 5, dans laquelle l'excipient pharmaceutique acceptable est choisi parmi les celluloses et les dérivés de la cellulose, le lactose, les amidons et les dérivés de l'amidon, les cyclodextrines, les polydextroses ou des mélanges desdites substances et est de préférence choisi parmi l'hydroxypropylcellulose, le lactose , la carmellose calcique et l'amidon prégélatinisé.

7. Forme posologique unitaire préparée à partir de la composition pharmaceutique granulée humide selon la revendication 5 ou 6, ladite forme posologique unitaire étant un comprimé ou une gélule, de préférence ladite forme posologique étant enrobée.

8. Composition pharmaceutique granulée humide selon la revendication 5 ou 6, dans laquelle ladite combinaison de tensioactifs est présente dans une plage de concentrations allant d'environ 0,1 % à 10 % (p/p) de ladite composition, de préférence dans la plage allant de 0,5 % à 5 % (p/p) de ladite composition.

9. Composition pharmaceutique granulée humide selon la revendication 5 ou 6, ladite composition comprenant également au moins un principe actif en plus du Candésartan cilexétil.

10. Composition pharmaceutique granulée humide selon la revendication 9, dans laquelle ledit principe actif additionnel est choisi dans le groupe constitué par les diurétiques, de préférence l'Hydrochlorothiazide, les agents antihypertenseurs, de préférence l'Amlodipine et ses sels, et les inhibiteurs de la réductase, de préférence l'Atorvastatine et ses sels.
